(19) Europäisches Patentamt
European Patent Office
Office européen des brevets

(11) Veröffentlichungsnummer: **0 137 339**
**B1**

(12) # EUROPÄISCHE PATENTSCHRIFT

(45) Veröffentlichungstag der Patentschrift: **18.03.87**

(51) Int. Cl.⁴: **C 07 K 7/06**, A 61 K 37/02

(21) Anmeldenummer: **84110911.9**

(22) Anmeldetag: **13.09.84**

(54) **Pharmakologisch aktive Peptide.**

(30) Priorität: **19.09.83 DE 3333752**

(43) Veröffentlichungstag der Anmeldung: **17.04.85 Patentblatt 85/16**

(45) Bekanntmachung des Hinweises auf die Patenterteilung: **18.03.87 Patentblatt 87/12**

(84) Benannte Vertragsstaaten: **AT BE CH DE FR GB IT LI LU NL SE**

(73) Patentinhaber: **Brantl, Victor, Dr. med., Dipl.-Chem., Frauenplatz 10, D-8000 München 2 (DE)**

(72) Erfinder: **Brantl, Victor, Dr. med., Dipl.- Chem., Frauenplatz 10, D-8000 München 2 (DE)**

(74) Vertreter: **Schacht, Wilhelm, Dr. jur., Am Eselsweg 47, D-6500 Mainz 1 (DE)**

(56) Entgegenhaltungen:
**DE-A-2 740 699**
**FR-A-2 465 713**
**US-A-4 254 024**

**Chemical Abstracts, Band 100, Nr. 13, 26. März 19 84, Columbus, Ohio, USA; V. SALVADORI et al "Opioid peptides. Structure-activity relationships in Beta-Ala dermorphin tetrapeptides", Seite 701, Spalte 1, Abstract Nr. 103859z;**
**Chemical Abstracts, Band 98, Nr. 11, 14. März 1983, Columbus, Ohio, USA; R. DE CASTIGLIONE et al. "Synthesis of dermorphin and Hyp-dermorphin, two opiate-like peptides from amphibian skin", Seite 604, Spalte 1, Abstract Nr. 89862b;**
**Chemical Abstracts, Band 95, Nr. 5, 3. August 1981, Columbus, Ohio, USA; R. DE CASTIGLIONE et al. "Synthesis of demorphins, a new class of opiate-like peptides", Seite 814, Spalte 1, Abstract Nr.**

(56) Entgegenhaltungen: (Fortsetzung)
**43634p;**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 137 339 B1

LIBER, STOCKHOLM 1987

**Beschreibung**

Aus der DT-OS 29 36 099 sind pharmakologisch aktive Peptide bekannt, welche insbesondere opiatartig wirken, die im Vergleich zu den Peptiden der DT-OS 29 21 216 stärkere opiatartige Wirkungen auslösen und eine höhere Stabilität gegenüber proteolytischen Enzymen (z.B. im Rattenplasma) aufweisen. Die Peptide der DT-OS 29 36 099 weisen folgende Grundstruktur auf:

$L\text{-}Tyr^1\text{-}X^2\text{-}L\text{-}Phe^3\text{-}L\text{-}Pro^4$-......

wobei L-Tyr= L-Tyrosin, X eine D-Aminosäure; L-Phe= L-Phenylalanin und L-Pro= L-Prolin bedeutet. Es sei bereits an dieser Stelle vermerkt, daß die <u>4. Aminosäure</u> nach der DT-DS 29 36 099 in der <u>L-Form</u> vorliegt; die D-Form ist nicht erwähnt. Es hat sich gezeigt, daß diese Peptide nur eine bestimmte maximale Wirkungstärke, insbesondere eine opiatartige, aufweisen (Vergl. Tab. 1, S. 20) Die DT-OS 30 34 897, die der DT-OS 29 36 099 sehr ähnlich ist beschreibt pharmakologisch aktive Peptide der folgenden Grundstruktur:

L-Tyr-A-L-Phe-B-......

wobei L-Tyr= L-Tyrosin, A eine D-Aminosäure, L-Phe= L-Phenylalanin und B ein neutraler <u>L-Aminosäurerest</u> ist (z.ß.-Glycin). Auch hier sei vermerkt daß der 4. Aminosäurerest in der <u>L-Form</u> vorliegen soll.

Es sei an dieser Stelle noch angemerkt, daß die Peptide der Europ. Anmeldung Nr- 81305519-1 (Publ Nr 0053029) im wesentl. ident. mit denjenigen der vorher bereits genannten DT-DS 2921216 sind. Die Europ. Anmeldung 81305519.1 besitzt zwar an der Position des 4. Aminosäurerestes des Peptids eine D-Aminosäure (D-Pro), aber es wurde nicht erkannt, daß eine besonders starke opiatartige Wirkung durch die Kombination eines D-Alaninrestes in der zweiten Position zusammen mit einem D-Prolinrest in der 4. Position erzielt wird. Zusätzlich weisen die Peptide der Europ. Anmeldung 8130519.1 den Nachteil auf, daß diese sehr schnell im Blut abgebaut werden und so nur kurz anhaltende pharmakologische Wirkungen am lebenden Organismus zeigen. (Dieser Abbau wird durch die Einführung des D-Alaninrestes in der zweiten Position verhindert). Hierzu sind insbesondere die genauen Daten aus der DT-OS 2921216 und der DT-OS 2936099 zu entnehmen.

Aufgabe der vorliegenden Erfindung ist es, neuartige pharmakologisch aktive Peptide zu schaffen, insbesondere opiatartig wirkend, die stärkere pharmakologische Wirkungen, inbesondere opiatartige und/oder höhere enzymatische Stabilität gegenüber peptidspaltenden Enzymen aufweisen.

Diese Aufgabe ist gemäß der Erfindung dadurch gelöst, daß die Peptide folgende Struktur aufweisen:

L-Tyr-X-L-Phe-X-T
L-Tyr-X-L-Phe-X-A-T
L-Tyr-X-L-Phe-X-A-X-T
L-Tyr-X-L-Phe-X-A-X-B-T

wobei L-Tyr für den N-terminalen Aminosäurerest L-Tyrosin, X für einen beliebigen Aminosäurerest der D-Konfiguration, L-Phe für den Aminosäurerest L-Phenylalanin stehen. A und B können beliebige Aminosäurereste sein können. T für OH, OR, $NH_2$, NHR, $NR_2$ oder NHNHR' steht, wobei R gegebenenfalls substit. linear. oder verzweigtes $C_{1\ 10}$-alkyl, Adamantyl, C $_{bis\ 10}$-Cycloalkyl oder $C_{6\text{-}8}$-Ar(alk)yl, zweckmäßigerweise Phenyl, Benzyl oder-Phenyläthyl bedeutet und R' Wasserstoff, lineares oder verzweigtes $C_{1\text{-}10}$-Alkyl, Cycloalkyl oder $C_{6\text{-}8}$-Aralkyl, $C_{2\text{-}8}$-Alkenyl, lineares, verzweigtes oder cyclisches aliphat. $C_{1\text{-}16}$-Acyl, gegebenenfalls durch OH, $NH_2$, $C_{1\text{-}4}$-Alkoxy oder Halogen substituiert, aromatisches Acyl, gegebenfalls durch OH, $NH_2$, Halogen oder $C_{1\text{-}4}$-Alkoxy substituiert; lineares verzweigtes oder cyclisches $C_{3\text{-}11}$ aliphatisches Urethan oder aromatisches Urethan darstellt und deren pharmazeutisch annehmbare Salze.

Die erfindungsgemäßen Peptide weisen in Gegensatz zu den im Stande der Technik beschriebenen Peptid eine stärkere opiatartige Wirkung und/oder eine erhöhte Stabilität gegenüber peptidspaltenden Enzymen auf (Vergl.Ausführungsbeispiel).

Nach einer Weiterbildung der Erfindung sind die Peptide dadurch gekennzeichnet, daß der Aminosäurerest X die Aminosäuren:

D-Alanin, D-Threonin, D-Serin, D-Methionin, D-Valin, D-Phenylalanin, D-Leucin, D-Isoleucin, D-Arginin, D-Histamin, D-Prolin, D-Hydroxyprolin, D-Lysin, D-Glutamin, D-Glutaminsäure, D-Asparagin oder, D-Asparaginsäure und A, B die aromatischen Aminosäuren Tyrosin oder Phenylalanin, oder Glycin bedeuten.

Die besonderen Eigenschaften der erfindungsgemäßen Peptide sind vermutlich ganz oder teilweise auf die besondere Anordnung der D-Aminosäurereste zurückzuführen. Es sind dies insbesondere diejenigen, an der 2. Position (vom Tyrosin ausgehend) und an der 4. Position, welche die hohe enzymat. Stabilität und hohe opiatartige Wirksamkeit bedingen. (Vergl. Ausführungsbeispiel)

Nach einer anderen Weiterbildung sind die Peptide dadurch gekennzeichnet, daß diese das N-terminale Tyrosin der Formel:

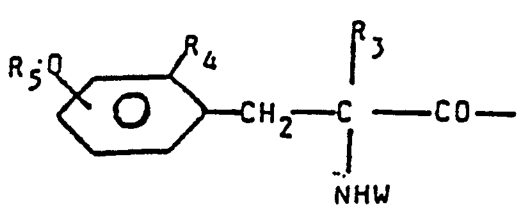

aufweisen, wobei
$R_3$ für Wasserstoff oder eine Alkylgruppe mit 1 bis 4 C-Atomen,
$R_4$ für Wasserstoff oder zusammen mit $R_3$ für eine Äthylenbrücke,
$R_5$ für Wasserstoff, eine Alkylgruppe mit 1 bis 4 C-Atomen oder eine $R_6CO$-Gruppe,
$R_6$ für einen gesättigten oder ungesättigten, geradkettigen oder verzweigten Alkylrest mit 1 bis 17 C-Atomen, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei-die $R_5O$-Gruppe sich in meta- oder para-Stellung zum

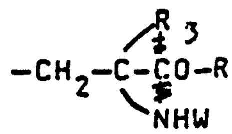
-Rest befindet,

W für Wesserstoff, Alkyl mit 1 bis 5 C-Atomen, Alkenyl mit 3 bis 5 C-Atomen, Cyclopropylmethyl, Cyclobutylmethyl, $R_6CO$-, stehen
b) das Phenylalanin in der allgemeinen Formel:

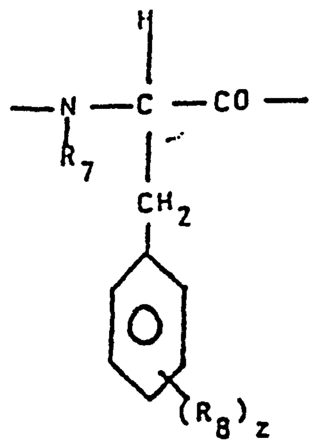

vorliegt, wobei
$R_7$ für Wasserstoff oder Alkyl mit 1 bis 4 C-Atomen,
$R_8$ für Wasserstoff, Fluor, Chlor, Brom, Nitro, Alkyl mit 1 bis 4 C-Atomen z für 1 oder 2 stehen.

Diese Weiterbildung hat den Vorteil, daß z.B. durch die Einführung von Halogenen wie Cl oder F am arom. Ring des Phenylalanins die Lipophilie der Stoffe so erhöht wird, daß diese vom Blut besser in das zentrale Nervensystem übertreten und so weniger Substanz für dieselbe Wirkung benötigt wird. Ebenso wirkt sich die Einführung einer N-Methylgruppe am Phenylalaninrest aus.

Die erfindungsgemäßen Peptide können nach einer Weiterbildung dadurch gekennzeichnet sein, daß die Aminosäurereste Alanin, Phenylalanin sowie A und B als Dehydroaminosäuren vorliegen.

Zur weiteren Erhöhung der enzymat. Stabilität kann das Phenylalin der 3. Position des Peptids in der D-Form vorliegen. Überraschenderweise hat dies keinen Verlust der opiatartigen Wirkung zur Folge und führt zu einer noch höheren enzymatischen Stabilität der Peptide.

Die erfindungsgemäßen Peptide zeigen starke Wirkungen auf das zentrale Nervensystem. Dies können insbesondere starke opiatartige (analgetische) Effekte wie auch kataleptische Wirkungen sein. Weiterhin können Wirkungen beobachtet werden, die als neuroleptikaartig zu bezeichnen sind.

Die erfindungsgemäßen Peptide weisen auch Wirkungen auf das cardiovaskuläre System auf. Dies kann insbesondere eine blutdrucksenkende Wirkung sein.

Die erfindungsgemäßen Peptide können in verschiedenen Formen verabreicht werden (z.B. Tabletten, oder in Lösung); die Applikationsart kann oral, parenteral (z.B. i.v.,i.m u. s.c), transdermal, nasal, vaginal oder rektal sein. Es können in den verschiedenen Zubereitungsformen Hilfsstoffe enthalten sein, die die Resorption begünstigen. Die Dosierung der aktiven Bestandteile einer pharmazeutischen Zubereitung kann entsprechend der unterschiedlichen Art der Verabreichung stark variieren. Allgemein kann ein Dosisbereich von 0.001 bis 100 mg Wirkstoff pro Kilogramm Körpergewicht angegeben werden, der dem Säugetierorganismus zugeführt werden muß, um einen therapeutischen Effekt, wie z.B. Analgesie zu erzielen.

Nach einer besonders vorteilhaften Ausbildung der Erfindung weisen die erfindungsgemäßen Peptide die folgenden Formeln auf:

Tyr-D-Ala-Phe-D-Ala-Tyr-$NH_2$
Tyr-D-Ala-Phe-D-Ala-Phe-$NH_2$
Tyr-D-Ala-Phe-D-Als-Tyr-D-Pro-$NH_2$
Tyr-D-Ala-Phe-D-Ala-Phe-D-Pro-$NH_2$
Tyr-D-Ala-Phe-D-Ala-Tyr-D-Pro-Ser-$NH_2$
Tyr-D-Ala-Phe-D-Ala-Phe-D-Pro-Ser-$NH_2$
Tyr-D-Ala-Phe-D-Ala-Tyr-D-Ala-Ser-$NH_2$
Tyr-D-Ala-Phe-D-Ala-Phe-D-Ala-Ser-$NH_2$
Tyr-D-Ser-Phe-D-Ala-Tyr-$NH_2$
Tyr-D-Ser-Phe-D-Ala-Phe-$NH_2$

**Ausführungsbeispiel**

Zunächst wird die Synthese zweier erfindungsgemäßer Peptide beispielhaft beschrieben, danach auf die pharmakologischen Eigenschaften dieser Peptide eingegangen.

Synthese zweier erfindungsgemäßer Peptide:

Die Synthese der beiden Peptide:
Tyr-D-Ala-Phe-D-Ala-Tyr-$NH_2$
Tyr-D-Ala-Phe-D-Als-Phe-$NH_2$
erfolgte so wie von Lottspeich et al. in Hoppe Seylers Z. Physiol. Chem., 361, 1835-1839 (1980) und in der DT-OS 29 36 099 beschrieben wurde. In einzelnen sei kurz auf die Syntheseschritte eingegangen.

Die einzelnen Aminosäurebausteine bzw. das Dipeptid stammen von der Bachem AG, Bubendorf, Schweiz oder von Fluka, Buchs, Schweiz.

Stufe I

a. Bildung des gemischten Anhydrids von Z-D-Ala 335 mg Z-D-Ala (1.5 mmol), Z= Benzoxycarbonylschutzgruppe, werden in 15 ml Dimethylformamid (DMF) unter Zusatz von N-Methylmorpholin gelöst und mit 180 µl (1.4 mmol) Chlorameisensäureisebutylester bei -15° C, 15 Minuten umgesetzt.

b. Vorbereitung der Aminokomponente 195 mg (1.0 mmol) Tyrosinmethylester oder 178 mg Phenylalaninmethylester werden in 20 ml DMF unter Zusatz von 110 µl (1.0 mmol) N-Methylmorpholin bei -15 C gelöst.

Nach Vorbereitung der Aminokomponente wird diese zu I a. zugesetzt und für 12 Stunden bei der o.g. Temperatur gehalten.

Vor der Aufarbeitung wird der 50%ige Überschuß an gemischten Anhydrid zerstört: Bei 0° C wird das pH des Reaktionsproduktes mit wässriger, gesättigter $KHCO_3$-Lösung auf pH 8 eingestellt und 30 min bei 0° C gerührt.

Danach wird das Dipeptid mit 50-100 ml Ethylacetat (Etac) extrahiert; das Etac/Peptidgemisch wird 5 x mit 20 ml gesättigter, wässriger Kochsalzlösung gewaschen. Nach zwei weiteren Waschvorgängen mit 10 ml Wasser wird die Etac-Phase eingedampft.

Die Abspaltung der Schutzgruppe erfolgt durch Hydrierung; hierzu wird das Dipeptid in 30 ml Methanol gelöst und 100 mg Palladium auf Aktivkohle (Merck, Darmstadt) zugegeben. Nach dem Verdrängen der Luft durch Stickstoff wird in das Reaktionsgefäß Wasserstoff eingeleitet. Die Hydrierung wird bei 25-30°C durchgeführt. Die Hydrierung ist beendet, wenn in wässriger Bariumhydroxidlösung kein Niederschlag mehr gebildet wird. Die Lösung wird filtriert, mit Wasser gewaschen und am Rotationsverdampfer einrotiert. Das verbleibende Zwischenprodukt wird dann wiederum als Aminokomponente im nächsten Kopplungeschritt eingesetzt.

Stufe II

a. Bildung des gemischtcn Anhydrids von Z-D-Ala-Phe 600 mg (1.6 mmol) Z-D-Ala-Phe werden wie unter Stufe I a. beschrieben behandelt.

b. Vorbereitung der Aminokomponente

Das in Stufe I gewonnene Dipeptid D-Ala-Tyr-OMet bzw. das analog gewonnene D-Al a-Phe-OMet wird wie unter 1 b. beschrieben gelöst und mit dem gemischten Anhydrid der Stufe II a. umgesetzt (12 Stunden bei -15° C).

Die Aufarbeitung, Extraktion und Hydrierung erfolgt wie in Stufe I beschrieben.

Als Endprodukt dieser Stufe wird das Tetrapeptid D-Ala-Phe-D-Ala-Tyr-OMet bzw. D-Ala-Phe-D-Ala-Phe-OMet erhalten.

Stufe III

a. Bildung des gemischten Anydrids von Z-Tyr 629 mg (1.4 mmol) Z-Tyr, 160 µ1 (1.4 mmol) N-Methylmorpholin und 170 µl (1.3 mmol) Chlorameisensäureisobutylester werden in 15 ml DMF wie vorher beschrieben gelöst.

b. Vorbereitung der Aminokomponente

Die Tetrapeptide der Stufe II (siehe oben) werden in 15 ml DMF gelöst und mit dem gemischten Anhydrid der Stufe III a. umgesetzt. Die weitere Prozedur und Aufarbeitung erfolgt wie bereits oben ausführlich beschrieben.

Stufe IV

Nach dem Abspalten der Schutzgrupppe werden die Peptidmethylester in üblicher Weise einer Ammonolyse unterworfen um diese in die entsprechenden Amide umzusetzen.

Stufe V

Die Peptide werden nach der Ammonolyse gereinigt, so wie von Lottspeich et al. in Hoppe Seylers Z. Physiol. Chem., 361, S. 1836 (1980) beschrieben. Das Syntheseendprodukt wird hierbei über eine Gel-Chromatographie gereinigt und sodann der Aminosäureanalyse zugeführt.

Die Aminosäureanalyse entsprach der Zusammensetzung der erfindungsgemäßen Peptide:

Tyr-D-Ala-Phe-D-Ala-Tyr-$NH_2$

Tyr-D-Ala-Phe-D-Ala-Phe-$NH_2$

Im folgenden Teil wird über die pharmakologischen Eigenschaften diser beiden beispielhaften erfindungsgemäßen Peptide berichtet.

Analog dem vorher beschriebenen Verfahren können weitere erfindungsgemäße Peptide folgender Formeln erhalten werden, wie zum Beispiel:

Tyr-D-Ala-Phe-D-Ala-Tyr-$NH_2$

Tyr-D-Ala-Phe-D-Ala-Phe-$NH_2$

Tyr-D-Ala-Phe-D-Ala-Tyr-D-Pro-$NH_2$

Tyr-D-Ala-Phe-D-Ala-Phe-D-Pro-$NH_2$

Tyr-D-Ala-Phe-D-Ala-Tyr-D-Pro-Ser-$NH_2$

Tyr-D-Ala-Phe-D-Ala-Phe-D-Pro-Ser-$NH_2$

Tyr-D-Ala-Phe-D-Ala-Tyr-D-Ala-Ser-$NH_2$

Tyr-D-Ala-Phe-D-Ala-Phe-D-Ala-Ser-$NH_2$

Tyr-D-Ser-Phe-D-Ala-Tyr-$NH_2$

Tyr-D-Ser-Phe-D-Ala-Phe-$NH_2$

Diese Peptide sind zum Teil noch stärker opiatartig wirksam und/oder weisen noch höhere Stabilität gegenüber peptidspaltenden Enzymen auf, als die in Tab. 1 beschriebenen beispielhaften Peptide III und IV.

**Biologische Wirkungen zweier beispielharter er findungsgemäßer Peptide.**

Die Tabelle 1 zeigt die pharmakologischen Eigenschaften zweier erfindungsgemäßer Peptide im Vergleich zu den Peptiden des Standes der Technik.

Als biologisches Testsystem zum Nachweis der opiatartigen Wirkung dient das Meerschweincheneileumpräparat (Detaillierte Darstellung vergl. DT-OS 29 21 216, Seite 24). Bei diesem Test wurden diejenigen Substanzkonzentrationen (nM) ermittelt, welche eine 50%ige Hemmung der elektrisch induzierten Kontraktion des Organpräparats bewirkten (sog. $IC_{50}$-Werte). Zusätzlich kann zum besseren Verständnis der $IC_{50}$-Wert für das Opiat Normorphin und Dermorphin (DT-OS 30 34 897) gleich 100% gesetzt werden und die relative Aktivität der einzelnen Substanzen im Vergleich zu Normorphin oder zu Dermorphin angegeben werden. Die Angabe der relativen Wirkungsstärke hat den Vorteil, daß so ev. Schwankungen in der Empfindlichkeit des Testsystems neutralisiert sind.

Die in der Tabelle 1 mit (*) gekennzeichnten Werte stimmen mit denjenigen überein, die von de Castiglione et al. beschrieben wurden (Peptides, 2, S. 266, Tab.1, Substanzen 1 und 3: Spalte CPI= guinea pig ileum, Meerschweinchendarmpräparat 1981.)

**TABELLE I**

| Nr. | Substanz | $IC_{50}$ | Rel. Wert Nor.=100 | Rel.Wert Der.=100 |
|---|---|---|---|---|
| - | Normorphin | 110 | 100 | 3.2 |
| - | Dermorphin | 3.5* | 3143 | 100 |
| I | Tyr-D-Ala-Phe-Pro-Tyr-$NH_2$ | 100 | 110 | 3.5 |
| 5 II | Tyr-D-Ala-Phe-Gly-Tyr-$NH_2$ | 7* | 1571 | 50 |
| III | Tyr-D-Ala-Phe-D-Ala-Tyr-$NH_2$ | 14 | 786 | 25 |
| IV | Tyr-D-Ala-Phe-D-Ala-Phe-$NH_2$ | 2.5 | 3667 | 140 |

$IC_{50}$-Werte (nM), vergl. Text, zweier beispielhafter erfindungsgemäßer Peptide (III und IV) in Vergleich zu denjenigen des Standes der Technik (Dermorphin, I, II) gemessen am isolierten Meerschweinchenileum. Die $IC_{50}$-Werte wurden aus 7 Einzelbestimmungen gemittelt; die Standardabweichung eines Einzelwertes vom Mittelwert ist kleiner als ± 14%. Die hemmende Wirkung der Substanz I, II, III, und IV konnte durch Zusatz des spezifischen Opiatantagonisten Naloxon aufgehoben werden (Endkonzentration im Organbad 900 nM); bei weiterem Zusatz von den Substanzen I bis IV in das Organbad (bei Gegenwart von Naloxon) konnte keine weitere Hemmung mehr beobachtet werden; die Hemmung der Substanzen I-IV ist somit als opiatspezifisch zu betrachten.

Die Tab. 1 zeigt, daß beide erfindungsgemäße Peptide III und IV im Vergleich zum Peptid I des St. d. T. (DT-OS 29 36 099) wesentlich stärker opiatartig wirksam sind, d.h. sie weisen kleinere $IC_{50}$-Werte auf. Das erfindungsgemäße Peptid IV weist im Vergleich zum Peptid II des St. d. T. (DT-OS 30 34 897) ebenfalls eine stärkere opiatartige Wirkung auf; das erfindungsgemäße Peptid III weist zwar keine höhere Aktivität als das Peptid II des St. d. T. auf, ist aber, wie im Ausführungsbeispiel beschrieben, wesentlich stabiler gegenüber peptidspaltenden Enzymen (als II) und somit besser für eine in vivo Verabreichung zur Erzielung pharmakologischer Wirkungen geeignet.

Die erfindungsgemäßen Peptide zeigen nicht nur in vitro sondern auch in vivo starke opiatartige Wirkungen (Analgesie). Als Testsystem diente der elektrisch stimulierte Rattenschwanz. Die Methode wurde so verwendet, wie von Brantl et al. 1981 beschrieben wurde (Life Sciences, 28, 1903-1909). Die Substanzen wurden intracerebroventrikulär verabreicht.

Die relative Wirksamkeit in vivo (Analgesie) der Substanzen der Tabelle 1 (mit Vergleichssubstanz Dermorphin) entsprach in etwa der relativen Wirkungsstärke, welche mit Hilfe des isolierten Meerschweinchenileums bestimmt wurde.

Enzymatische Stabilität der erfindungsgemäßen Peptide

Je 1 mg der Peptide Dermorphin, I, II, III und IV (Tab. 1) wurden in 1 ml frisch zubereiteter Krebs-Ringer Lösung gelöst und mit je 50 µg α-Chymotrypsin (Serva, Heidelberg; in 50 µl Krebs-Ringer Lösung gelöst) vermischt; unmittelbar nach dem Vermischen wurden je 100 µl entnommen, sofort auf 95°C erhitzt und für 15 Minuten auf dieser Temperatur gehalten (Das Erhitzen diente zur Inaktivierung des Enzyms). Die zu Beginn der Inkubation entnommene Probe dient als Ausgangswert (0-Wert). Die verbleibenden 950 µl des Peptid/Enzymgemisches wurden dann 5 Stunden lang bei 37°C inkubiert; der Inkubationsvorgang wurde dann ebenfalls durch Erhitzen auf 95°C beendet.

Nach Zentrifugation wurden gleiche Aliquots der jeweiligen Überstände quantitativ auf ihre opiatartige Wirkung, im Vergleich zum 0-Wert untersucht; als Nachweissystem für die opiatartige Wirkung diente wiederum das Meerschweinchenileum (wie vorher beschrieben). Es zeigte sich, daß die opiatartige Wirkung in den Inkubationsansätzen mit Dermorphin und dem Peptid II im Vergleich zum 0-Wert nach 5 stündiger Inkubationsperiode fast völlig verschwunden war, während hingegen Peptide III und IV ihre volle Opiataktivität beibehielten, d.h. diejenige Hemmung des Meerschweinchenileum-Präparats, die durch ein Aliquot des 0-Wertes erzielt wurde entsprach der gleichen Hemmung, die durch ein Aliquot gleichen Volumens des 5-stündigen Inkubationsansatzes erzeugt wurde. Die Substanz I zeigt eine höhere Stabilität als die Substanz II, ist aber auch wesentlich weniger stark opiartig wirksam als die erfindungsgemäßen Peptide III und IV. Insgesamt zeigt sich, daß die Einbringung einer D-Aminosäure an der Position 4 der bekannten Peptide I und II besonders vorteilhaft für die Erzielung einer besonders starken opiatartigen Wirkung ist und/oder um Peptide zu erhalten, die eine besonders hohe Stabilität gegenüber peptidspaltenden Enzymen aufweisen; Peptide, welche zur Erzielung pharmakologischer Wirkungen verabreicht werden unterliegen ja, wie allgemein bekannt ist, einem sehr raschen Metabolismus, durch peptidspaltende Enzyme, der ihre Verwendungsmöglichkeiten als Pharmaka stark einschränkt.

**Beispiel für eine pharmazeutische Formulierung**

(Tablette)

Substanz III (Tab 1) 140 mg
Lactose 480 mg
Maisstärke 20 mg
Polyvinylpyrrolidon (PVP) 10 mg
Magnesiumstearat 10 mg

Der Wirkstoff wird zusammen mit Lactose und Maisstärke gemischt und mit einer Lösung von PVP (50 %ig in wässrig. Äthanol) granuliert. Das Granulat wird getrocknet; nach Zusatz von Magnesiumstearat wird die Mischung in üblicher Weise zu einer Tablette gepresst.

**Patentansprüche**

1) Pharmakologisch aktive Peptide der Formel:
L-Tyr-X-L-Phe-X-T
L-Tyr-X-L-Phe-X-A-T
L-Tyr-X-L-Phe-X-A-X-T
L-Tyr-X-L-Phe-X-A-X-B-T
wobei L-Tyr für dén N-terminalen Aminosäurerest L-Tyrosin, X für einen beliebigen Aminosäurerest der D-Konfiguration, L-Phe für den Aminosäurerest L-Phenylalanin stehen, A und B beliebige Aminosäurereste sein können, T für OH, OR, $NH_2$, NHR, $NR_2$ oder NHNHR' steht, wobei R gegebenenfalls substit. linear. oder verzweigtes $C_{1-10}$-Alkyl, Adamantyl; $C_{bis\ 10}$-Cycloalkyl oder $C_{6-8}$-Ar(alk)yl, zweckmäßigerweise Phenyl, Benzyl oder Phenyläthyl bedeutet und R' Wasserstoff, lineares oder verzweigtes $C_{1-10}$-Alkyl, Cycloalkyl oder $C_{6-8}$-Aralkyl, $C_{2-8}$-Alkenyl, lineares verzweigtes oder cyclisches aliphat. $C_{1-16}$-Acyl, gegebenenfalls durch OH, $NH_2$, $C_{1-4}$-Alkoxy oder Halogen substituiert, aromatisches Acyl, gegebcnfalls durch OH, $NH_2$, Halogen oder $C_{1-4}$-Alkoxy substituiert; lineares, verzweigtes oder cyclisches $C_{3-11}$aliphatisches Urethan odcr aromatisches Urethan darstellt, und deren pharmazeutisch annehmbare Salze.

2) Pharmakologisch aktive Peptide nach Anspruch 1, dadurch gekennzeichnet, daß der Aminosäurerest X D-Alsnin, D-Threonin, D-Serin, D-Methionin, D-Valin, D-Phenylalanin, D-Leucin, D-Isoleucin, D-Arginin, D-Histamin, D-Prolin, D-Hydroxyprolin, D-Lysin, D-Glutamin, D-Glutaminsäure, D-Asparagin oder D-Asparaginsäure und A, B die aromatischen Aminosäuren Tyrosin oder Phenylalanin, oder Glycin bedeuten.

3) Pharmakologisch aktive Peptide nach Anspruch 1 und 2, dadurch gekennzeichnet, daß
a) das N-terminale L-Tyrosin der allgemeinen Formel:

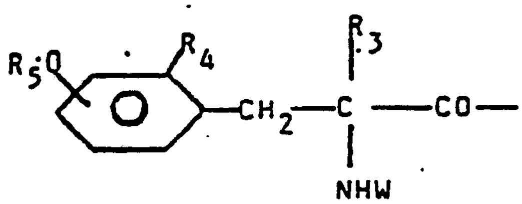

vorliegt, wobei
$R_3$ für Wasserstoff oder eine Alkylgruppe mit 1 bis 4 C-Atomen,
$R_4$ für Wasserstoff oder zusammen mit $R_3$ für eine Äthylenbrücke,
$R_5$ für Wasserstoff, eine Alkylgruppe mit 1 bis 4 C-Atomen oder eine $R_6CO$-Gruppe,
$R_6$ für einen gesättigten oder ungesättigten, geradkettigen oder verzweigten Alkylrest mit 1 bis 17 C-Atomen, einen Phenylrest oder einen Phenylalkylrest mit 7 bis 12 C-Atomen stehen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei die $R_5O$-Gruppe sich in meta- oder para-Stellung zum

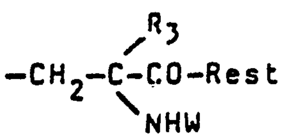
-Rest befindet,

W für Wasserstoff Alkyl mit 1 bis 5 C-Atomen, Alkenyl mit 3 bis 5 C-Atomen, Cyclopropylmethyl, Cyclobutylmethyl, $R_6CO$-, steht
b) das Phenylalanin der allgemeinen Formel vorliegt:

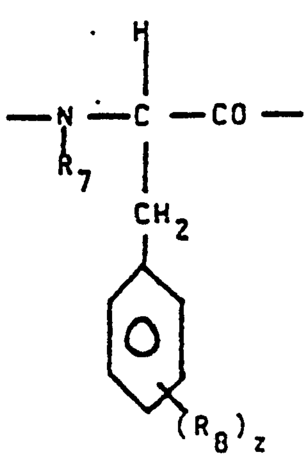

wobei

$R_7$ für Wasserstoff oder Alkyl mit 1 bis 4 C-Atomen,

$R_8$ für Wasserstoff, Fluor, Chlor, Brom, Nitro, Alkyl mit 1 bis 4 C-Atomen, z für 1 oder 2 stehen.

4) Pharmakologisch aktive Peptide nach einem der Ansprüche 1-3, dadurch gekennzeichnet, daß die Aminosäuren Alanin, Phenylalanin sowie A und B als Dehydroaminosäuren vorliegen.

5) Pharmakologisch aktive Peptide nach Anspruch 1-4, dadurch gekennzeichnet, daß das Phenylalanin in der 3. Aminsäureposition des Peptids (vom N-terminalen Ende gerechnet) in der D-Form vorliegt.

6) Pharmakologisch aktive Peptide nach einem der Ansprüche 1-5, dadurch gekennzeichnet, daß diese starke Wirkungen auf das zentrale Nervensystem ausüben.

7) Pharmakologisch aktive Peptide nach Anspruch 1-6, dadurch gekennzeichnet, daß die Peptide starke opiatartige Wirkungen in vivo und in vitro auslösen.

8) Pharmakologisch aktive Peptide nach Anspruch 1-5, dadurch gekennzeichnet, daß diese starke Wirkungen auf das Herz- und Kreislaufsystem ausüben.

9) Pharmazeutische Zubereitungen, die als aktiven Bestandteil eine oder mehrere der in den Ansprüchen 1-8 beschriebenen Peptide enthalten.

10) Pharmakologisch aktive Peptide nach Anspruch 1-3, dadurch gekennzeichnet, daß die Peptide folgende Struktur aufweisen:

Tyr-D-Ala-Phe-D-Ala-Tyr-$NH_2$
Tyr-D-Ala-Phe-D-Ala-Phe-$NH_2$
Tyr-D-Ala-Phe-D-Ala-Tyr-D-Pro-$NH_2$
Tyr-D-Ala-Phe-D-Ala-Phe-D-Pro-$NH_2$
Tyr-D-Ala-Phe-D-Ala-Tyr-D-Pro-Ser-$NH_2$
Tyr-D-Ala-Phe-D-Ala-Phe-D-Pro-Ser-$NH_2$
Tyr-D-Ala-Phe-D-Ala-Tyr-D-Ala-Ser-$NH_2$
Tyr-D-Ala-Phe-D-Ala-Phe-D-Ala-Ser-$NH_2$
Tyr-D-Ser-Phe-D-Ala-Tyr-$NH_2$
Tyr-D-Ser-Phe-D-Ala-Phe-$NH_2$

## Claims

1. Pharmacologically active peptides of the following formulas:

L-Tyr-X-L-Phe-X-T
L-Tyr-X-L-Phe-X-A-T
L-Tyr-X-L-Phe-X-A-X-T
L-Tyr-X-L-Phe-X-A-X-B-T

wherein L-Tyr is the N-terminal amino acid residue L-tyrosine, X is any amino acid of the D-form, L-Phe is the amino acid residue L-phenylalanine. A and B may represent any amino acid residue. T represents OH, OR, $NH_2$, NHR,$NR_2$, or NH NHR; at which R has, if necessary, the following meaning: a substituted linear or branched $C_{1-10}$-alkyl, adamantyl, $C_{1-10}$-cycloalkyl or $C_{6-8}$-aralkyl, suitable phenyl, benzyl or means phenylethyl and R'hydrogen, linear or branched $C_{1-10}$-alkyl, cycloalkyl or $C_{6-8}$-aralkyl, $C_{2-8}$-alkenyl, linear, branched or cyclic aliphatic $C_{1-16}$-acyl, if appropriate substituted by OH, $NH_2$, $C_{1-4}$-alkoxy or halogen, or $C_{1-4}$-alkoxy; linear, branched or cyclic $C_{3-11}$-aliphatic urethan or aromatic urethan and their pharmaceutically acceptable salts.

2. Pharmacologically active peptides according to claim 1 characterized in that the amino acid residue X is replaced by: D-alanine, D-threonine, D-serine, D-methionine, D-valine, D-phenylalanine, D-leucine, D-isoleucine, D-arginine,D-histamine, D-proline, D-hydroxyproline, D-lysine, D-glutamine, D-glutamic acid, D-asparagine, D-aspartic acid and A, B are replaced by the aromatic amino acids tyrosine and phenylalanine or glycine.

3. Pharmacologically active peptides according to claim 1 and 2 characterised in that the N-terminal L-tyrosine is of the general formula:

a)

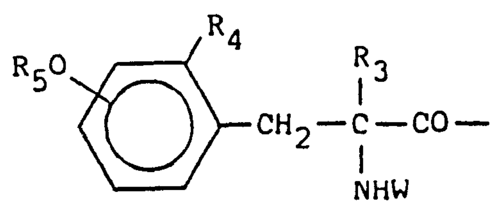

wherein means

$R_3$ for hydrogen or an alkyl group with 1 to 4 C-atoms,

$R_4$ for hydrogen or together with $R_3$ for an ethylene bond,

$R_5$ for hydrogen, an alkyl group with 1 to 4 C-atoms or a $R_6CO$-group,

$R_6$ for a saturated or unsaturated linear or branched alkyl residue with 1 to 17 C-atoms, a phenyl residue or a phenyl-alkyl residue with 7 to 12 C-atoms, wherein the phenyl residues can be substituted by 1 or 2 substituents from the halogen series, alkyl with 1 to 4 C-atoms or alkoxy with 1 to 4 C-atoms, wherein the $R_5O$-group is in the meta position or para position to

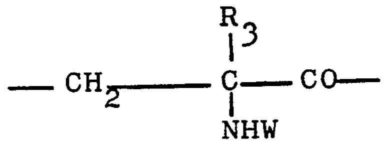

W for hydrogen alkyl with 1 to 5 C-atoms, alkenyl with 3 to 5 C-atoms, cyclopropylmethyl, cyclobutylmethyl, $R_6CO$-,

b) the phenylalanine of the general formula:

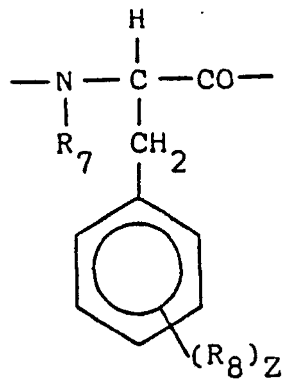

is substituted by:

$R_7$ for hydrogen or alkyl with 1 to 4 C-atoms,

$R_8$ for hydrogen, fluorine, chlorine, bromine, nitro, alkyl with 1 to 4 C-atoms for alkoxy with 1 to 4 C-atoms

Z for 1 or 2 substituents.

4. Pharmacologically active peptides according to one of the claims 1-3 characterized in that the amino acids alanine, phenylalanine as well as A and B are dehydro amino acids.

5. Pharmacologically active peptides according to one of the claims 1-4 characterized in that the phenylalanine in the 3rd amino acid position of the peptide (calculated from the N-terminal end) is of the D-form.

6. Pharmacologically active peptides according to one of the claims 1-5 characterized in that they exhibit strong effects on the central nervous system.

7. Pharmacologically active peptides according to claim 1-6 characterized in that the peptides exhibit in vivo and in vitro strong opiate-like effects.

8. Pharmacologically active peptides according to claim 1-5 characterized in that they exhibit strong effects on the cardiovascular system.

9. Pharmacologically active peptides according to claim 1-8 characterized in that the peptides have the following structures

Tyr-D-Ala-Phe-D-Ala-Tyr-$NH_2$
Tyr-D-Ala-Phe-D-Ala-Phe-$NH_2$
Tyr-D-Ala-Phe-D-Ala-Tyr-D-Pro-$NH_2$
Tyr-D-Ala-Phe-D-Ala-Phe-D-Pro-$NH_2$
Tyr-D-Ala-Phe-D-Ala-Tyr-D-Pro-Ser-$NH_2$
Tyr-D-Ala-Phe-D-Ala-Phe-D-Pro-Ser-$NH_2$
Tyr-D-Ala-Phe-D-Ala-Tyr-D-Ala-Ser-$NH_2$
Tyr-D-Ala-Phe-D-Ala-Phe-D-Ala-Ser-$NH_2$
Tyr-D-Ser-Phe-D-Ala-Tyr-$NH_2$
Tyr-D-Ser-Phe-D-Ala-Phe-$NH_2$

10. Pharmaceutical preparations, which contain one or more of the peptides described in claim 1-9.

**Revendications**

1. Peptides à activité pharmacologique des formules suivantes:
L-Tyr-X-L-Phe-X-T
L-Tyr-X-L-Phe-X-A-T
L-Tyr-X-L-Phe-X-A-X-T
L-Tyr-X-L-Phe-X-A-X-B-T
dans lesquelles
L-Tyr est le résidu d'amino-acide N-terminal L-tyrosine, X est un résidu d'amino acide quelquonque de la configuration stéréochimique D, L-Phe est le résidu d'amino acide L-phénylalanine. A et B peuvent être chaques quelconques résidus d'amino acides. T est OH, OR,$NH_2$, NHR, $NR_2$ ou NHNHR dans lesquels R, si necessaire, est
- un alkyle $C_{1-10}$, adamantyle qui sont linéaires ou ramifiés et substitués;
- un cycloalkyle $C_{1-10}$ ou un aralkyle $C_{6-8}$, avantageusement un phényle, benzyle, ou phénylethyle; et R'est
- un atome d'hydrogène,
- un alkyle $C_{1-10}$ linéaire ou ramifié,
- un cyclalkyle ou un aralkyle $C_{6-8}$,
- un groupe alkényle ayant de 2 à 8 atomes de carbone, substitué par un groupement hydroxy, amino, alcoxy $C_{1-4}$ ou un atome d'halogène,
- acyle aromatique, si necessaire substitué par OH, $NH_2$, halogène ou alcoxy $C_{1-4}$
- un groupe de type méthane linéaire, ramifié ou cyclique $C_{3-11}$ aliphatique,
- un groupe de type uréthane aromatique et
les sels pharmaceutiquement acceptables des substances que l'on vient de mentionner.

2. Peptides à activité pharmacologique selon la revendication 1, caractérisés en ce que le résidu d'amino acide X est D-alanine, D-threonine, D-serine, D-methionine, D-valine, D-phénylalanine, D-leucine, D-isoleucine, D-arginine, D-histamine, D-proline, D-hydroxyproline, D-lysine, D-glutamine, acide de D-glutamine, D-asparagine, acide de D-asparagine; A et B représentent les amino acides aromatiques tyrosine et phénylalanine ou glycine.

3. Peptides à activité pharmacologique suivant l' une des revendications 1 et 2, caractérisés en ce que
a) la L-tyrosine N-terminale de formule générale

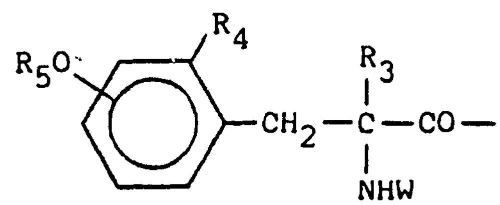

est present. En ce formule générale représente $R_3$ un atome d'hydrogène ou un groupe alkyle en $C_1$ à $C_4$,
$R_4$ un atome d'hydrogène ou forme avec $R_3$ un pont éthylène,
$R_5$ un atome d'hydrogène, un groupe alkyle en $C_1$ à $C_4$ ou un groupe $R_6CO$-,
$R_6$ un résidu d'alkyle saturé ou non saturé, linéaire ou ramifié comortant 1 à 17 atomes de carbone, un reste phényle ou phénylalkyle comportant 7 à 12 atomes de carbone, les restes phényles pouvant être substitués par 1 ou 2 substituants de la série des halogènes, des restes alkyles en $C_1$ à $C_4$ ou d'alcoxy en $C_1$ à $C_4$, le groupe $R_5O$ étant en position méta- ou para- par rapport au reste

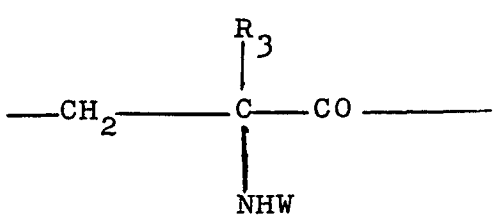

et
W étant un atome d'hydrogène, un alkyle $C_1$ à $C_5$, un alkényle en $C_3$ à $C_5$, un cyclopropylméthyle, un cyclobutylméthyle, ou un groupe $R_6CO$-.
b) la phénylalanine est de formule générale

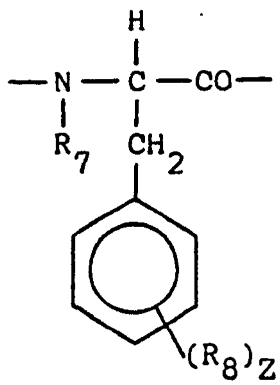

dans laquelle

$R_7$ est un atome d'hydrogène ou un groupe alkyle en $C_1$ à $C_4$,

$R_8$ est un atome d'hydrogéne, - de fluor, - de chlore, - de brome, un groupe nitro, un groupe alkyle en $C_1$ à $C_4$ et

z représente 1 ou 2 substituents.

4. Peptides à activité pharmacologique, suivant l'une des revendications 1 - 3, caractérisés en ce que les amino acides alanine, phénylalanine, et A et B sont dehydro-amino acides.

5. Peptides à activité pharmacologique, suivant l,une des revendications 1 - 4, caractérises en ce que le phénylalanine en position troixième d'amino acide du peptide (calculé de bout N-terminal) est présent dans la configuration stéréochimique D.

6. Peptides à activité pharmacologique, suivant l'une des revendications 1 - 5, caractérisés en ce qu'ils faient des effets vigoureux en système nerveux central.

7. Peptides à activité pharmacologique, suivant l'une des revendications 1 - 6, caractérisés en ce que les peptides montrent des effets vigoureux opiacés in vivo et in vitro.

8. Peptides à activité pharmacologique, suivant l'une des revendications 1 - 5, caractérisés en ce qu'ils montrent des effets vigoureux en système cardiovasculaire.

9. Peptides à activité pharmacologique suivant l'une des revendications 1 - 8 en ce que les peptides ont des structures suivantes:

Tyr-D-Ala-Phe-D-Ala-Tyr-$NH_2$
Tyr-D-Ala-Phe-D-Ala-Phe-$NH_2$
Tyr-D-Ala-Phe-D-Ala-Tyr-D-Pro-$NH_2$
Tyr-D-Ala-Phe-D-Ala-Phe-D-Pro-$NH_2$
Tyr-D-Ala-Phe-D-Ala-Tyr-D-Pro-Ser-$NH_2$
Tyr-D-Ala-Phe-D-Ala-Phe-D-Pro-Ser-$NH_2$
Tyr-D-Ala-Phe-D-Ala-Tyr-D-Ala-Ser-$NH_2$
Tyr-D-Ala-Phe-D-Ala-Phe-D-Ala-Ser-$NH_2$
Tyr-D-Ser-Phe-D-Ala-Tyr-$NH_2$
Tyr-D-Ser-Phe-D-Ala-Phe-$NH_2$

10. Preparations pharmaceutiques quelles contenent un ou plusieurs des peptides décrits en revendications 1 - 9.